# EUROPEAN PATENT APPLICATION

(11) **EP 2 354 792 A1**
(43) Date of publication of application: **10.08.2011**
(21) Application number: 10152930.3
(22) Date of filing: 08.02.2010
(51) Int. Cl.: G01N 33/564, G01N 33/68

(54) **Method for detecting anti-drug antibodies**

(71) Applicant: Biomonitor A/S, 2100 Copenhagen Ø (DK)
(72) Inventor: Svenson, Morten, 2100 Copenhagen Ø (DK); Bovin, Lone Frier, 2830 Virum (DK)
(74) Representative: Simonsen, Jan Lyngberg

(57) **Abstract**

The present inventions relates to the monitoring or assaying of anti-bio-agent antibodies, such as anti-drug antibodies (ADA) in patients who may have developed an antibody response in treatments with immunoglobulin bio-agents.

## Description

### FIELD OF THE INVENTION

The present inventions relates to the monitoring or assaying of anti-bio-agent antibodies, such as anti-drug antibodies (ADA) in patients who may have developed an antibody response in treatments with immunoglobulin bio-agents.

### BACKGROUND OF THE INVENTION

According to the Pharmaceutical Research and Manufacturers of America (PhRMA) millions of people have benefited from medicines and vaccines developed through biotechnology, and according to recent reports there are numerous further biopharmaceuticals for the treatment of more than 100 diseases currently in development. In their survey, the PhRMA identifies 324 biotechnology medicines in development for nearly 150 diseases. These include 154 medicines for cancer, 43 for infectious diseases, 26 for autoimmune diseases and 17 for AIDS/HIV and related conditions. These potential medicines, all of which are either in human clinical trials or under review by the Food and Drug Administration, will bolster the list of 108 biotechnology medicines already approved and available to patients.

The widespread use of biopharmaceuticals raises the possibility that some patients may develop antibodies to the drugs, which can greatly decrease the efficacy of the (biopharmaceutical) drug, or completely obliterate the benefit of taking the drug, resulting in considerable wasted expenditure on ineffective therapy, and more importantly, lost time in the treatment of the disorder which can have catastrophic effects in terms of the development of disease and disorders in the patient. Indeed, response failure due to induction of antibodies (Abs) against biopharmaceuticals is increasingly being realized. The development of host antibodies can be remedied by increasing dosage - although this is typically a delayed and rather temporary response as the prescription dosage is typically only increased once patient symptoms noticeably deteriorate, and the increased dosage may well result in further augmentation of the patients immune system. There is therefore a need for methods to determine the bioavailability of biopharmaceutical drugs.

The development of host (patient) antibodies against biopharmaceutical use is particularly of issue when the drug is delivered chronically, i.e. periodic administration over a period of months or years.

Anti-TNF alpha drugs are among important group of this type of biopharmaceuticals.

Anti-tumor necrosis factor (TNF) therapy has become an important alternative in the management of several chronic immunoinflammatory diseases. Three recombinant anti-TNF drugs are currently approved for clinical use in patients with various chronic inflammatory diseases such as rheumatoid arthritis, Crohn's diseases and severe psoriasis: 1) Remicade™ (infliximab), a mouse-human IgG1-kappa anti-TNF-alpha monoclonal antibody, 2) Enbre™ (etanercept), a fusion protein of human TNF receptor 2 and human IgG1, and 3) Humira™ (adalimumab), a fully human IgG1-kappa anti-TNF-alpha monoclonal antibody. Two other anti-TNF-alpha antibody constructs have shown promise in pivotal phase III trials in patients with some of the same diseases: 4) Cimzia™ CDP870 (certolizumab pegol), a PEGylated Fab fragment of a humanized anti-TNF-alpha monoclonal antibody, and 5) CNTO 148 (golimumab), a fully human IgG1-kappa anti-TNF-alpha monoclonal antibody. All of these proteins dramatically lower disease activity and, in some patients, may induce remission. Unfortunately, however, not all patients respond favorably to anti-TNF antibodies. Some patients either do not respond at all (primary response failure) or they respond initially but have later relapses (secondary response failure) despite increased dosage and/or more frequent administration of the drugs. The reason(s) for these response failures are not always clear but interindividual and even intraindividual differences in bioavailability and pharmacokinetics may contribute to the problem. Immunogenicity of the drugs causing patients to develop anti-antibodies is a problem now recognized by many investigators, drug-controlling agencies, health insurance companies and drug manufacturers. Monitoring of patients for circulating levels of functional anti-TNF drugs and anti-antibody development is therefore warranted so that administration can be tailored to the individual patient and so that prolonged therapies can be provided effectively and economically with little or no risk to the patients.

By way of a non limiting example, we refer to Infliximab (Remicade®).

Infliximab is a mouse-human chimeric monoclonal IgG antibody against tumor necrosis factor-alpha (TNF-alpha), in combination with methotrexate, is approved for the treatment of moderate-to-severe rheumatoid arthritis (RA) in patients who have an inadequate response to one or more disease-modifying antirheumatic drugs (DMARD). In randomized clinical trials, intravenous infusions of infliximab, 3 mg/kg every 4 to 8 weeks, induce a positive response at 30 weeks in approximately 55% of patients and the response can be maintained in many patients with repeated infusions.

With repeated infusions, however, the formation of neutralizing anti-infliximab antibodies becomes a problem requiring increased doses or more frequent drug administration and may necessitate discontinuation of therapy because of secondary response failure and/or infusion-related side effects; this has been observed in both RA patients and in patients with other immunoinflammatory diseases. Our own clinical experience, for example, rapidly showed that the generally recommended dosage of 3 mg/kg at weeks 0, 2, 6 and every 8 weeks thereafter was inadequate in a large proportion of patients. The mean weekly dosages per patient of infliximab at 3 year follow up were 35 (n=5), 54 (n=35), 44 (n=26), and 38 (n=17) mg at years 2002, 2003, 2004, and 2005, respectively. Furthermore, the recommended dose regimen was originally established on the basis of clinical trials using relatively large cohorts of RA patients of both sexes, with differences in age, co-morbidities and concurrent therapies. In clinical practice, however, patients with RA or any other chronic inflammatory disease treated with infliximab may differ considerably from the average patient in randomized clinical trials. For example, even though the initial bioavailability of infliximab approaches 100% because of the intravenous administration of the drug, differences in pharmacokinetics may result in individual patients having inadequate drug levels for extended periods of time between infusions. This problem can be exaggerated by the appearance of antibodies. Indeed, response failures are frequent, and development of assays that can be used to monitor bioavailability and Ab development is of direct clinical importance.

There is therefore a problem with the use of biopharmaceuticals that the patient's immune system can develop an antibody response, and this problem can result in ineffective patient treatment and increased costs of treatment.

A number of studies have reported a concentration-effect relationship of therapeutic proteins directed against TNF-alpha in patients with RA and Crohn's disease and an inverse relation between drug levels and ADA.

Different methods have been used to assess circulating levels of ADA, such as anti-TNF biopharmaceuticals. Some of these are based on enzyme immunoassays (EIA) where the anti-TNF biopharmaceuticals are immobilized on plastic beads or wells and bridging the binding of labeled biopharmaceutical by ADA is used as readout. Other assays detect complexes of ADA and biopharmaceutical by selective absorption ex. by the binding of Fab of an immunoglobuline biopharmaceutical to protein A, or to antibodies to anti-light chain Fab not expressed by the biopharmaceutical.

Certain embodiments of the present invention are disclosed, by the same inventors as the present invention, in Bendtzen et al., Arthritis & Rheumatism Vol 54, No 12, published December 2006, which is hereby incorporated by reference.

The present invention provides a highly effective and sensitive assay for detection of the *in vivo* ADA against bio-agents, such as a very important class of biopharmaceuticals, monoclonal antibodies and chrimeric constructs expressing Fc of human immunoglobulines.

### OBJECT OF THE INVENTION

It is an object of embodiments of the invention to provide an easier, more specific and more reliable method often with improved sensitivity in the measurement and/or detection of host derived antibodies recognising a specific bio-agent, such as host derived antibodies that has been developed as an immunological response to the biopharmaceutical use of the bio-agent.

It is further an object of embodiments of the invention to provide an assay that is highly suitable for high throughput screenings (HTC).

### SUMMARY OF THE INVENTION

It has been found by the present inventor(s) that improved methods for the detection and/or quantification of host derived antibodies recognising a specific bio-agent may be provided by a method comprising the steps of contacting the biological sample potentially containing an ADA with an antibody binding agent, such as protein G on a solid support, and thereafter contacting the complex formed with a labeled specific bio-agent recognized by the ADA, with the subsequent quantification of labeled bio-agent.

So, in a first aspect the present invention relates to a method for detecting the presence of or for the measurement of the amount of specific antibodies in a biological sample derived from a subject, which antibodies recognise a specific bio-agent, the method comprising the sequential steps of:
a) contacting the biological sample with an antibody-binding agent under conditions that allow binding of the specific antibodies recognising the specific bio-agent, if present, to the antibody-binding agent to form a first complex;
b) contacting the first complex, if present, with the specific bio-agent in a form which comprises a first detectable label; to form a second complex;
c) measuring a signal from the first detectable label present in the complex formed in step b) to detect the presence of or to measure the amount of antibodies which recognise the bio-agent in the biological sample.

In a second aspect the present invention relates to a kit comprising:
a) An antibody-binding agent;
b) A bio-agent in a form which comprises a first detectable label,
c) Optionally a first unspecific blocking agent and/or a second blocking agent;
d) Optionally a suitable reagent for detection of said first or second detectable label.

In a third aspect the present invention relates to a method of treatment of a disease in a patient being treated with a biopharmaceutical bio-agent, said method comprising performing the method according to the invention on a sample derived from the patient to determine whether the patient requires either an altered dosage regime of the biopharmaceutical or an alternative pharmaceutical therapy.

### LEGENDS TO THE FIGURE

Fig. 1 is a schematic illustration of the principle of the assay for determining ADA against human monoclonal Ab constructs.
Fig. 2. Comparing ADA measurements by RIA and EIA. Illustrative results of running test of the same positive Infliximab ADA sera in EIA (Fig 2a) at 0.25% and in RIA (Fig. 2b) at 1% concentration.
Fig. 3 illustrates background activity and cross-reactivity of ADA against Infliximab with two other anti-TNFa constructs tested by substituting labelled Infliximab/Remicade with labelled Humira or Enbrel.

### DETAILED DISCLOSURE OF THE INVENTION

### Definitions

The term "bio-agent" refers to any biological compound, such as a biopharmaceutical compound, which may elicit an anti-drug antibody response in a patient receiving the biopharmaceutical compound, such as any protein therapeutic. In some embodiments the "bio-agent" refers to a monoclonal antibody, such as a humanized or fully human monoclonal antibody. The bio-agent may also be protein constructs comprising fragments of immunoglobulin, such as etanercept. In some embodiments the "bio-agent" refers to either a single light chain biopharmaceutical or a single light chain biodiagnostic. The bio-agent may consists of an intact light chain immunoglobulin, or a fragment thereof which comprises at least a variable domain, and at least part of the light chain constant region. The bio-agent may be free of heavy chain immunoglobulins. Table 1 provides a list of bio-agents which comprise of monoclonal antibodies, including those whose anti-drug antibody response may be determined using the methods of the present invention. In some embodiments, the bioagent refers to an allergen. Accordingly, the method according to the invention may be used to measure the concentration of circulating, host-derived allergen-specific antibodies in a subject, such as in human serum or plasma.

The heavy chain of antibodies typically has a molecular weight of approximately 50 kDa, whereas the light chains typically have a molecule weight of approximately 25 kDa. The light and heavy chains are joined together by a disulfide bond near the carboxyl terminus of the light chain. The heavy chain is divided into an Fc portion, which is at the carboxyl terminal (the base of the Y), and a Fab portion, which is at the amino terminal (the arm of the Y). Carbohydrate chains are attached to the Fc portion of the molecule. The Fc portion of the Ig molecule is composed only of heavy chains. The Fc region contains protein sequences common to all Igs as well as determinants unique to the individual classes. These regions are referred to as the constant regions because they do not vary significantly among different Ig molecules within the same class. The Fab portion of the Ig molecule contains both heavy and light chains joined together by a single disulfide bond. One heavy and one light chain pair combine to form the antigen binding site of the antibody. Human light chain antibodies can be of either lambda or kappa isotypes.

The term "intact light chain" refers to a polypeptide which consists of both one or more variable regions and constant regions (or part thereof) of a light chain isotype polypeptide. The intact light chain is the product of the expression of a light chain encoding polynucleotide, taking into account post-translational modifications which may occur during production within the expression system.

The term "biological sample" or "sample" refers to a sample which is obtained or derived from a patent which comprises patient derived immunoglobulin and may therefore be referred to as an immunoglobulin sample. By way of example, the sample may be selected from the group consisting of blood, blood serum, lymph fluid, lymph node tissue, spleen tissue, bone marrow, or an immunoglobulin enriched fraction derived from one or more of these tissues. In a preferred embodiment the sample is, or comprises blood serum or is an immunoglobulin enriched fraction derived from blood serum or blood. In one embodiment the sample is, or is derived from, a bodily fluid. In one embodiment the sample is derived (obtained) from body tissue. In some embodiments, the sample is obtained form a subject who has been exposed to the bio-agent, such as repeatedly exposed to the same bio-agent. In some embodiments, the sample is obtained form a subject who has not recently been exposed to the bio-agent. In one embodiment, the sample is obtained form the subject prior to the planned administration of the bio-agent.

The term "specific antibody" or "specific antibodies" as used herein refers to a plurality of antibodies derived from a biological sample of a subject that specifically recognise a particular bio-agent. It may be a plurality of antibodies within the same class or isotypes of antibodies, such IgG, IgE, IgA, IgD, and IgM. Accordingly in some embodiments the specific antibodies being detected or measured is within a particular isotype of antibodies, such as IgG and/or IgE. Typically the specific antibodies of the biological sample have not been purified with respect to any specific component, such as specific antibodies of the biological sample.

The term "subject" refers to the individual from which the biological sample is taken. Typically the subject is a patient who is either: (i) being considered for treatment, or undergoing treatment, or previously received treatment, wherein the treatment involves the administration of a monoclonal antibody based biopharmaceutical (bio-agent), or (ii) is being considered for diagnosis, or undergoing diagnosis, or has previously undergone diagnosis for a disorder or a disease, wherein the diagnosis involves the administration bio-agent is used to specifically detect and/or localise the presence of the disorder or disease or disease causing agent. The patient may be an animal, such as a mammal, preferably a human being.

When referred to "measurement of the amount of antibodies" herein, it refers to the determination of the concentration of host-derived antibody against the bio-agent in the subject (such as in the sample, or tissue corresponding to the sample). By comparing to data of the known concentration of anti-drug antibodies (ADA), the concentration of anti-drug antibodies (ADA) can be determined in samples with unknown concentration.

The method of the invention allows for the measurement of *in vivo* amount of host derived immunoglobulin molecules present in the subject (or biological sample), which immunoglobulin molecules recognise a particular bio-agent, such as in the subject having received a particular bio-agent as a biopharmaceutical for en extended period of time. Such methods may be qualitative (i.e. presence of absence), or quantitative.

The method according to the invention may, for example, be used for identifying primary non- or low-responders, e.g. for treatment. These may, for example, be patients that happen to have an innate or a pre-developed immunoglobulin response to the bio-agent. Where the bio-agent is a diagnostic antibody, the identification of primary non- or -low responders can ensure the selection of a suitable diagnostic agent for each individual patient.

The method according to the invention may, for example, be used for identifying patients with secondary response failure. Secondary response failures can be asymptomatic, i.e. the only symptoms are that the treatment has become less effective or even non-effective. In this instance the use of the method according to the invention can be used to identify the development of secondary response failure before the patient or medical practitioner has noticed that the treatment is less effective. A higher dosage of treatment may be applied to ensure the correct and effectively *in vivo* concentration is achieved, or an alternative treatments can be selected, or a combination thereof. When the bio-agent is a diagnostic, the development of secondary response failure can be particularly catastrophic. Radio-labelled monoclonal antibodies are routinely used in the monitoring of diseases such as cancers, and some infectious diseases, where it is important to determine the size and/or location of the disease/agent - for example in identifying the presence/location of any secondary metastases. When the development of response failure (either primary or secondary) occurs unnoticed, the patient may be given the 'all clear' - *i.e*. a false negative result, this can lead to the cessation of treatment and the latter re-appearance of the disease, often in a far more developed and possibly untreatable condition.

A further category of response failure is the development of (e.g. secondary) response failure associated with adverse side effects. Although rare, the development of a host-immune response in a subject can be accompanied by deleterious or unpleasant side effects. These may be caused by the development of antibodies which recognise the human or humanised bio-agents, but may then fail to distinguish with other host immunoglobulins. The present invention can therefore be used to prevent the administration of bio-agents to subjects who have either an innate or have previously developed an immune response to the bio-agent, subjects who may, for example, be vulnerable to adverse side effects associated with response failure.

The kit according to the invention is typically accompanied by instructions for use in the method according to the invention. The bio-agent or biopharmaceutical can be as according to those described herein.

Clearly one major application area for the method of the present invention is in the selection and management of treatment regimes which involve the administration of monoclonal biopharmaceuticals to patients. Therefore, the method for determining the concentration or amount of ADA, as described herein, can be incorporated into a method of treatment of a disease or a disorder. By monitoring of the immunological status of the subject using the methods of the invention during the course of therapeutic treatment, the selection and/or administration of the biopharmaceutical agent can be tailored to ensure maximum therapeutic benefit to the patient, whilst ensuring cost effective use of expensive biopharmaceutical agents.

The method according to the invention may be used to determine whether the patient requires either an altered dosage regime of the biopharmaceutical or alternative pharmaceutical therapy.

Suitably the method may involve a periodic assessment of the serum concentration of ADA in the patient.

The invention provides for a method of determining whether the lack of treatment response in a patient is due to the formation of patient derived immunoglobulins against the bio-agent.

The invention provides for a method of selecting the appropriate drug treatment for a patient suffering from a disease which is treatable with a bio-agent (using the method steps referred to herein).

The invention provides for a prognostic method for the determination of the likelihood of whether a patient will develop secondary response failure to bio-agent (using the method steps referred to herein).

"Suitable solid supports" as used herein include any material that is an insoluble matrix and has a rigid or semi-rigid surface to which the antibody-binding agent can be linked or attached. Exemplary solid supports include, but are not limited to, substrates such as nitrocellulose, polyvinylchloride, polypropylene, polystyrene, latex , polycarbonate, nylon, dextran, chitin, sand, silica, pumice, agarose, cellulose, glass, metal, polyacrylamide, silicon, rubber, polysaccharides, polyvinyl fluoride; diazotized paper; activated beads, magnetically responsive beads, and any materials commonly used for solid phase synthesis, affinity separations, purifications, hybridization reactions, immunoassays and other such applications. The support can be particulate or can be in the form of a continuous surface and includes membranes, mesh, plates, pellets, slides, disks, capillaries, hollow fibers, needles, pins, chips, solid fibers, gels (e.g. silica gels) and beads, (e.g., pore-glass beads, silica gels, polystyrene beads optionally cross-linked with divinylbenzene, grafted co-poly beads, polyacrylamide beads, latex beads, dimethylacrylamide beads optionally crosslinked with N-N'-bis-acryloylethylenediamine, iron oxide magnetic beads, and glass particles coated with a hydrophobic polymer. In a some embodiments, the antibody-binding agent is attached to a microtiter plate or to a population of magnetic bead.

The terms "label," "labeled," "detectable label," and "detectably labeled" refer to a molecule capable of detection, including, but not limited to, radioactive isotopes, fluorophores, luminescers, chemiluminescers, enzymes, enzyme substrates, enzyme cofactors, enzyme inhibitors, chromophores, dyes, metal ions, metal sols, ligands (e.g., biotin or haptens), fluorescent nanoparticles, gold nanoparticles, and the like. The term " fluorophore" refers to a substance or a portion thereof that is capable of exhibiting fluorescence in the detectable range. Particular examples of labels that can be used include, but are not limited to fluorescein, rhodamine, dansyl, umbelliferone, Texas red, luminol, acridinium esters, NADPH, beta-galactosidase, horseradish peroxidase, glucose oxidase, alkaline phosphatase and urease. The label can also be an epitope tag (e.g., a His- His tag), an antibody or an amplifiable or otherwise detectable oligonucleotide.

### Labelling of bio-agents:

Numerous methods of labelling immunoglobulins are known in the art and can be used for the purposes of the present invention.

In one embodiment, the bio-agent is labelled by incubating the bio-agent with a labelled antigen, wherein the antigen is the specific antigen recognised by the bio-agent when used inside the body.

A preferred label is a radio label, which allow the labelled bio-agent to be used in radio-immuno assays. For example, ¹²⁵I labelling of Infliximab is described in Svensen et al., J.Clin Invest, 92, 2533-2539.

It is envisaged that by use of highly sensitive mass detection techniques, such as MALTI-TOF analysis, possibly in conjunction with immunoglobulin purification techniques, that the presence of the bio-agent - host immunoglobulin complex could be detected without the use of an exogenous label. For example, by using size exclusion chromatography, the free bio-agent and the host-immunoglobulin/probe associated bio-agent can be separated. The host-immunoglobulin associated bio-agent/probe (complex) can then be denatured (or a marker peptide from the bio-agent can be isolated), and the amount of 'free' bio-agent (i.e. not associated with host-immunoglobulin) and complexed bio-agent can then be compared by suitable protein quantification methods, such as by use of an immuno assay, or by MALDI-TOF, for example. Therefore, in one embodiment, it is not required to obtain an exogenously labelled bio-agent as the physical (chemical/immunological features (for example) of the bioagent itself may be used as a label.

However, for ease of use, it is considered that the use of an exogenously labelled bio-agent is preferred - suitably the exogenous label is a distinct chemical or physical entity, not present in the unlabelled bio-agent, e.g. which may be incorporated into the bio-agent (such as a radio-labelled amino-acid), or is conjugated or otherwise attached to the bio-agent once the bio-agent has been prepared (e.g. a fluorescent or luminescent label).

In some embodiment the labelled bioagent is an ¹²⁵I labelled anti-TNF-alpha bio-agent, such as those described herein.

In other embodiments the bio-agent is labelled with biotin and used in conjunction with labelled avidin/streptavidin complex to strengthen the signal to noise ratio of the specific detection signal.

### Incubating the biological sample with the antibody-binding agent and labelled bio-agent:

As a first step of the method according to the invention, the biological sample is contacted and incubated with an antibody-binding agent under conditions that allow binding of the antibodies recognising the specific bio-agent, if present, to the antibody-binding agent. With this step a first complex is formed. Typically incubations are performed in a suitable media, such as an assay buffer. Incubation may occur in a fluid phase, or on a solid phase. Preferably the incubations are performed on a solid phase, such in an ELISA plate.

As antibody-binding agent any compound or protein may be used that more or less specifically binds to antibodies. The antibody-binding agent may be selected from the list consisting of anti-Ig, suchs as Fc-specific or Fab specific antibodies, protein G, Protein A, Protein H, Protein L, and Protein A/G fusion protein. Accordingly, the antibody-binding agent may be selected to specifically bind the particular subtype of ADA, such as when using Protein A to bind with high affinity to human IgG1 and IgG2. The antibody-binding agent may also be selected not to bind a particular subtype of host-derived antibodies, so as to lower the binding of non-relevant host-derived antibodies.

Once the first complex is formed and a labelled bio-agent has been obtained, the labelled bio-agent is incubated with the first complex, typically in a suitable media, such as an assay buffer. The labelled bio-agent is typically purified prior to incubation, e.g. by size-exclusion or molecular size-chromatography. Again in this step, incubation may occur in a fluid phase, or on a solid phase. When in a liquid phase, both the labelled bio-agent and first complex are typically present in the fluid phase, such as in an assay buffer.

In some embodiments, the first and/or second complex may be attached to a solid phase, such as an affinity matrix/column support or magnetic bead. This facilitates a fractionation step, as the host immunoglobulin bound to antibody-binding agent and subsequently to labelled bio-agent, which can easily be separated or fractionated by e.g. affinity chromatography or use of a magnet (referring to affinity matrix/support and magnetic bead embodiments, respectfully). Alternatively attachment of the complexes to a (dense) particle, such as a bead can allow fractionation by, e.g. centrifugation or filtration.

One interesting embodiment is where the probe is biotinylated, and step c) involves the capture of the biointylated immunoglobulin complex by an avidin/strptavidin bead, which can subsequently be isolated /fractionated) e.g. by use of a magnet (and magnetic beads) or other types of beads, such as those referred to herein.

Both liquid and solid phase assays are typically performed in the presence of a blocking agent, such as milk proteins or BSA to prevent or reduce non-specific binding. Also blocking may be performed with non-labelled non-relevant immunoglobulin molecules. Conditions for blocking, such as concentrations, time and temperatures of incubations are well known for the persons skilled in the art.

The method according to the invention may benefit from some optimization dependent on amount of specific antibodies in the sample as well as total amount of immunoglobulin. Accordingly in some embodiments it is advantageous to measure the total amount of immunoglobulin in the biological sample prior to the assay in order to determine if maximal absorption to the antibody-binding agent has been reached. The signal derived from the specific antibodies recognising the bio-agent in the biological sample may be correlated and/or corrected according to the total amount of immunoglobulin in the sample. Also amount of biological sample applied to the antibody-binding agent may be adjusted dependent on total amount of immunoglobulin and amount of specific antibodies.

In one embodiment the assay is performed in a fluid phase, such as a fluid phase radioimmunoassay.

In one embodiment the assay is performed in a solid phase immunoassay, such as using ELISA.

*Potential subsequent isolation of a fraction which is enriched for the complex with labelled bio-agent:*

As described herein, the separation of the labelled bio-agent may be performed using numerous methods known in the art, typically based on molecular affinity:

As referred to above, in one embodiment, the method comprises the steps of (i) incubating the biological sample with an antibody-binding agent and (ii) incubating this formed complex with the labelled bio-agent. In such embodiments, the complexes formed is typically attached to a solid support which allows for the isolation of the fraction which is enriched for the complex and labelled bio-agent. For instance, the complex of biological sample with an antibody-binding agent may be attached to an affinity matrix as part of an affinity chromatography step - e.g. using an affinity column. Alternatively, the complex of antibody-binding agent and antibody recognising the specific bio-agent may be attached to a bead, such as a magnetic bead, for example. As well as (dense) beads and magnetic beads, fluorescent, luminescent or coloured beads may also be used - these can be sorted using, for example, FACS.

*Measuring a signal from the detectable label present on the bio-agent.*

The signal detected from the complexes formed in step c) or fractions obtained there from can only be derived from complexes formed wherein host immunoglobulin has recognised/is bound to the labelled bio-agent.

The detection of the signal is therefore a measure of the level of host immunoglobulins present in the sample which recognise/binds to the bio-agent.

Typically, step c) comprises a comparison step where data from one or more control sample(s) are used, which allows calibration of the data of the signal referred to in step c) to the data obtained from samples where the concentration of host-immunoglobulins which bind to the bio-agent are known.

### Suitable bio-agents and disorders

An extensive list of monoclonal antibody therapeutics in clinical development and approved products are disclosed in the 2006 PhRMA Report entitled '418 Biotechnology Medicines in Testing Promise to Bolster the Arsenal Against Disease'. It is considered that the present invention may be used against these as well as other single-light chain monoclonal antibodies used as therapeutics or in vivo diagnostics. See table 1 for examples of monoclonal antibodies, which have either been approved or are currently in development.

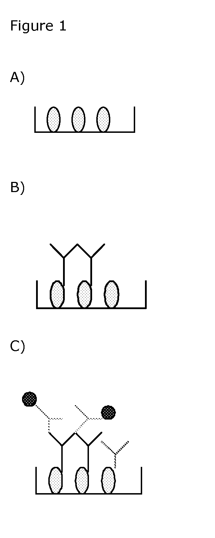

Particularly preferred bio-agents are the anti-TNFalpha monoclonal antibodies, which include (see Figure 1) Remicade™ (infliximab), a mouse-human IgG1-kappa anti-TNF-alpha monoclonal antibody, 2) Enbrel™ (etanercept), a fusion protein of human TNF receptor 2 and human IgG1, and 3) Humira™ (adalimumab), a fully human IgG1-kappa anti-TNF-alpha monoclonal antibody. Two other anti-TNF-alpha antibody constructs have shown promise in pivotal phase III trials in patients with some of the same diseases: 4) Cimzia™ CDP870 (certolizumab pegol), a PEGylated Fab fragment of a humanized anti-TNF-alpha monoclonal antibody, and 5) CNTO 148 (golimumab), a fully human IgG1-kappa anti-TNF-alpha monoclonal antibody.

A preferred class of bio-agents are anti-TNF-alpha single chain monoclonal antibodies which are used in treatment of numerous autoimmune diseases, such as - rheumatoid arthritis, juvenile idiopathic arthritis, ankylosing spondylitis (Bechterew's disease), inflammatory bowel diseases (Crohn's diseases and ulcerative colitis), severe psoriasis, chronic uveitis, severe sarcoidosis and Wegener's granulomatosis.

Whilst is it recognised that the present invention is particularly useful in determining the concentration of ADA against anti-TNF-alpha alpha single chain monoclonal antibodies - it is clear that the present invention is suitable for use in the determination of the bioavailability/concentration of any biopharmaceutical, which may be used within the body, such as for therapeutic or diagnostic purposes. Table 1 provides a list of medical indications which correlated to various monoclonal bio-agents used in vivo.

The present invention can therefore be use in a method of treatment where the treatment (or diagnosis) comprises administering a single chain monoclonal antibody to the subject. Suitably the method can be for the treatment or diagnosis of one or more of the disorders/diseases referred to herein, including one or more of the following:
Infectious diseases, such as respiratory syncytial virus (RSV), HIV, anthrax, candidiasis, staphylococcal infections, hepatitis C

Autoimmune diseases, such as rheumatoid arthritis, Crohn's disease, B-cell non hodgkin's lymphoma, Multiple scleorisis, SLE, ankylosing spondylitis, lupus, psoriatic arthritis, erythematosus.

Inflammatory disorders such as rheumatoid arthritis (RA), juvenile idiopathic arthritis, ankylosing spondylitis (Bechterew's disease), inflammatory bowel diseases (Crohn's diseases and ulcerative colitis), severe psoriasis, chronic uveitis, sarcoidosis, Wegener's granulomatosis, and other diseases with inflammation as a central feature.

Blood disorders, such as sepsis, septic shock, paroxysmal nocturnal hemoglobinuria, and hemolytic uremic syndrome.

Cancer, such as colorectal cancer, non-Hodgkin's lymphoma, B-cell chronic lymphocytic leukemia, anaplastic large-cell-lymphoma, squamous cell cancer of the head and neck, treatment of HER2-overexpressing metastatic breast cancer, acute myeloid leukemia, prostate cancer (e.g. adenocarcinoma), small-cell lung cancer, thyroid cancer, malignant melanoma, solid tumors, breast cancer, early stage HER2-positive breast cancer, first-line non-squamous NSCLC cancers, AML, hairy cell leukemia, neuroblastoma, renal cancer, brain cancer, myeloma, multiple myeloma, bone metastases, SCLC, head/neck cancer, first-line pancreatic, SCLC, NSCLC, head and neck cancer, hematologic and solid tumors, advanced solid tumors, gastrointestinal cancer, pancreatic cancers, cutaneous T-cell lymphoma, non-cutaneous T-cell lymphoma, CLL, ovarian, prostate, renal cell cancers, mesothelin-expressing tumors, glioblastoma, metastatic pancreatic, hematologic malignancies, cutaneous anaplastic large-cell MAb lymphoma, AML, myelodysplastic syndromes.

Cardiovascular disease, such as atherosclerosis acute myocardial infarction, cardiopulmonary bypass, angina.

Metabilic disorders such as diabetes, such as type-1 diabetes mellitus

Digestive disorders, such as Crohn's disease, C. difficile disease, ulcerative colitis

Eye disorders such as uveitis.

Genetic Disorders such as paroxysmal nocturnal hemoglobinuria (PNH)

Neurological Disorderssuch as osteoarthritis pain and Alzheimer's disease.

Respiratory Disorders such as respiratory diseases, asthma, chronic obstructive pulmonary disorders (COPD, nasal polyposis, pediatric asthma.

Skin diseases, such as psoriasis, including chronic moderate to severe plaque psoriasis.

Transplant rejection, such as acute kidney transplant rejection, reversal of heart and liver transplant rejection, prevention of renal transplant rejection, prophylaxis of acute kidney transplant rejection, renal transplant rejection.

Other disorders, such as diagnosis of appendicitis, kidney inflammation postmenopausal osteoporosis (bone disorders), hypereosinophilic syndrome, eosinophilic esophagitis and peanut allergy.

In one embodiment the disease is selected from one or more of the above groups or specific diseases/disorder. Preferred diseases are diseases where repeated dosages of the bio-agent are used, such as autoimmune diseases.

**Table 1: Therapeutic and diagnostic monoclonal antibodies (Approved are underlined)**

| Product Name | Sponsor | Indication |
|---|---|---|
| Infectious diseases | | |
| | | |
| Synagis® palivizumab | Medlmmune | prevention of respiratory syncytial virus (RSV) |
| anti-HIV-1 MAb | Polymun Scientific | HIV infection treatment |
| | *Vienna, Austria* | |
| CCR5 MAb | Human Genome Sciences | HIV infection |
| | *Rockville, MD* | |
| Cytolin® | CytoDyn | HIV infection |
| anti-CD8 MAb | *Santa Fe, NM* | |
| NM01 | SRD Pharmaceuticals | HIV infection |
| | *Los Ange*/*es, CA* | |
| PRO 140 | Progenics Pharmaceuticals | HIV infection |
| | *Tarrytown, NY* | |
| TNX | 355 Tanox MAb HIV infection Phase II | 355 Tanox MAb HIV infection Phase II |
| ABthrax™ raxibacumab | Human Genome Sciences | anthrax |
| | | |
| Anthim™ (ETI-204) (Orphan Drug) | Elusys Therapeutics | anthrax |
| anti-hsp90 MAb | NeuTec Pharma | candidiasis |
| anti-staph MAb | MedImmune | Prevention of staphylococcal infections |
| Aurexis tefibazumab | Inhibitex | prevention and treatment of S. *aureus* bacteremia |
| bavituximab | Peregrine Pharmaceuticals | hepatitis C treatment |
| MDX-1303 | Medarex PharmAthene | anthrax |
| Numax™ motavizumab | MedImmune | RSV |
| Tarvacin™ bavituximab | Peregrine Pharmaceuticals | hepatitis C |
| XTL 6865 | XTL Biopharmaceuticals | hepatitis C |

| Autoimmune disorders | | |
|---|---|---|
| Product Name | Sponsor | Indication |
| Humira® adalimumab | Abbott Laboratories | rheumatoid arthritis |
| Remicade™ infliximab | Centocor | Crohn's disease, rheumatoid arthritis |
| Rituxan® ritiximab | Genentech Biogen Idec | B-cell non hodgkin's lymphoma, relapse in patients following rituxan treatment. Rheumatoid arthritis |
| Tvsarbi® natalizumab | Biogen Idec | Multiple scleorisis |
| ABT 874 | Abbott Laboratories | multiple sclerosis, |
| Actemra | Roche | rheumatoid arthritis, |
| AME 527 | Applied Molecular | rheumatoid arthritis |
| AMG 108 | Amgen | rheumatoid arthritis |
| | | |
| AMG 714 | Amgen | rheumatoid arthritis |
| anti-CD16 MAb | MacroGenics | immune thrombocytopenic |
| CNTO 1275 | Centocor *Horsham, PA* | multiple sclerosis |
| daclizumab (anti-CD25 MAb) | PDL BioPharma *Fremont, CA* Biogen Idec *Cambridge, MA* | multiple sclerosis (see also respiratory) |
| denosumab (AMG 162) | Amgen *Thousand Oaks, CA* | rheumatoid arthritis |
| | | |
| ETI-201 | Elusys Therapeutics *Pine Brook, NJ* | SLE |
| golimumab | Centocor *Horsham, PA* | rheumatoid arthritis |
| HuMax-CD20 (ofatumumab) | Genmab *Princeton, NJ* | rheumatoid arthritis |
| Humira® adalimumab | Abbott Laboratories | ankylosing spondylitis juvenile rheumatoid arthritis |
| HuZAFT™ fontolizumab | PDL BioPharma *Fremont, CA* Biogen Idec *Cambridge, MA* | rheumatoid arthritis |
| IMMU-106 (hCD20) | Immunomedics *Morris Plains, NJ* | autoimmune disease |
| LymphoStat-B™ belimumab | Human Genome Sciences *Rockville, MD* | rheumatoid arthritis, SLE |
| MEDI-545 (MDX-1103) | Medarex *Princeton, NJ* MedImmune *Gaithersburg, MD* | lupus |
| MLN 1202 | Millennium Pharmaceuticals *Cambridge, MA* | multiple sclerosis |
| ocrelizumab (2nd anti-CD20) (R1594) | Genentech *South San Francisco, CA* Biogen Idec *Cambridge, MA* Roche *Nutley, NJ* | rheumatoid arthritis |
| OKT3-gamma-1 | Johnson & Johnson Pharmaceutical Research & Development *Raritan, NJ* | psoriatic arthritis |
| Rituxan® rituximab | Genentech *South San Francisco, CA* Biogen Idec *Cambridge, MA* | rheumatoid arthritis (DMARD inadequate responders), lupus, primary progressive multiple sclerosis, SLE (see also cancer) relapsing-remitting multiple sclerosis |
| TRX 1 | TolerRx | cutaneous lupus |
| (anti-CD4) | *Cambridge, MA* | erythematosus |

| Blood disorders | | |
|---|---|---|
| ReoPro® | Centocor | anti-platelet prevention of blood clots |
| abciximab | Eli Lilly | (PTCA), angina (PTCA) |
| urtoxazumab | Teijin Pharma | hemolytic uremic |
| Afelimomab | Abbot Laboratories | Sepsis, septic shock |
| Eculizumab | Alexion Pharmaceuticals | Paroxysmal nocturnal hemoglobinurea. |

| Cancer | | |
|---|---|---|
| Product Name | Sponsor | Indication |
| Avastin™ bevacizumab | Genentech | metastatic colorectal cancer |
| Bexxar® tositumomab, iodine I 131 tositumomab | GlaxoSmithKline | non-Hodgkin's lymphoma |
| Campath® alemtuzumab | Berlex Laboratories Genzyme | B-cell chronic lymphocytic leukemia |
| Erbitux™ cetuximab | Bristol-Myers Squibb Medarex | colorectal cancer squamous cell cancer of the head and neck |
| Herceptin® trastuzumab | Genentech | treatment of HER2-overexpressing metastatic breast cancer |
| Mylotarg™ qemtuzumab ozoqamicin | Wyeth | Acute myeloid leukemia |
| OncoScint® CR/OV satumomab pendetide | CYTOGEN | detection, staging and follow-up of colorectal cancers |
| ProstaScint® capromab pentetate | CYTOGEN | detection, staging and follow-up of prostate adenocarcinoma |
| Rituxan® ritiximab | Genentech Biogen Idec | B-cell non hodgkin's lymphoma, relapse in patients following rituxan treatment. |
| Verluma® nofetumomab | DuPont Pharmaceuticals | detection of small-cell lung cancer |
| Zevalin™ ibritumomab tiuxetan | IDEC Pharmaceuticals | Non-hodgkin's lymphoma |
| 1311-huA33 | Life Science Pharmaceuticals *Greenwich, CT* | colorectal cancer |
| 1D09C3 | GPC Biotech *Waltham, MA* | relapsed/refractory B-cell lymphomas |
| AGS-PSCA MAb | Agensys *Santa Monica, CA* Merck *Whitehouse Station, NJ* | prostate cancer |
| | | |
| AMG 102 | Amgen Thousand Oaks, CA | cancer |
| AMG 479 | Amgen Thousand Oaks, CA | cancer |
| AMG 623 | Amgen Thousand Oaks, CA | B-cell chronic lymphocytic leukemia (CLL) (see also autoimmune) |
| AMG 655 | Amgen Thousand Oaks, CA | cancer |
| AMG 706 | Amgen Thousand Oaks, CA | imatinib-resistant GIST, advanced thyroid cancer |
| anti-CD23 MAb | Biogen Idec Cambridge, MA | CLL |
| anti-CD80 MAb | Biogen Idec Cambridge, MA | non-Hodgkin's B-cell lymphoma |
| anti-idiotype cancer vaccine | Viventia Biotech Toronto, Ontario | malignant melanoma |
| anti-lymphotoxin beta receptor MAb | Biogen Idec Cambridge, MA | solid tumors |
| anti-PEM MAb | Somanta Pharmaceuticals Irvine, CA | cancer |
| anti-Tac(Fv)-PE38 immunotoxin | National Cancer Institute Bethesda, MD | leukemia, lymphoma |
| | | |
| Avastin® bevacizumab | Genentech *South San Francisco, CA* | relapsed metastatic colorectal cancer first-line metastatic breast, first-line non-squamous NSCLC cancers |
| AVE 9633 maytansin-loaded anti-CD33 MAb | sanofi-aventis *Bridgewater, NJ* | AML |
| bavituximab | Peregrine Pharmaceuticals *Tustin, CA* | solid cancers (see also infectious) |
| CAT 3888 | Cambridge Antibody Technology | hairy cell leukemia |
| chimeric MAb | National Cancer Institute | neuroblastoma |
| CNTO 328 | Centocor | renal cancer |
| Cotara™ | Peregrine Pharmaceuticals | brain cancer |
| bivatuzumab | Boehringer Ingelheim Pharmaceuticals *Ridgefield, CT* | cancer |
| CP-751,871 | Pfizer | multiple myeloma |
| CS 1008 | Daiichi Sankyo Sankyo Pharma Development *Parsippany, NJ* | cancer |
| BrevaRex™ antibody-based immunotherapy | Vi Rexx *Edmonton, Alberta* | breast cancer, multiple myeloma |
| denosumab | Amgen | bone loss induced by hormone ablation therapy for breast or prostate cancer, prolonging bonemetastases-free survival (see also autoimmune, other) bone metastases in breast cancer |
| ecromeximab | Kyowa Hakko USA | malignant melanoma |
| EMD 273063 | EMD Lexigen | solid tumors malignant melanoma, neuroblastoma, SCLC |
| Erbitux™ | Bristol-Myers Squibb | head/neck cancer, first-line pancreatic, first-line NSCLC,- second-line NSCLC, first line colorectal, second-line colorectal cancers |
| GMK | Progenics Pharmaceuticals | prevention of recurrence following surgery to remove primary melanoma in high-risk patients |
| Campath® alemtuzumab | National Cancer Institute *Bethesda, MD* Berlex Laboratories *Montville, NJ* | leukemia, lymphoma |
| | | |
| Herceptin® trastuzumab | Genentech *South San Francisco, CA* | early stage HER2-positive breast cancer first-line metastatic HER2-positive breast cancer in combination with Taxotere® |
| HGS-ETR1 | Human Genome Sciences *Rockville, MD* | hematologic and solid tumors |
| HGS-ETR2 (mapatumumab) | Human Genome Sciences *Rockville, MD* | hematologic and solid tumors |
| HGS-TR2J | Human Genome Sciences *Rockville, MD* | advanced solid tumors |
| HuC242-DM4 | ImmunoGen *Cambridge, MA* | colorectal, gastrointestinal, NSCLC, pancreatic cancers |
| HuMax-CD4 (zanolimumab) | Genmab *Princeton, NJ Serono Rockland, MA* | cutaneous T-cell lymphoma non-cutaneous T-cell lymphoma |
| HuMax-CD20 (ofatumumab) | Genmab *Princeton, NJ* | CLL, non-Hodgkin's lymphoma (see also autoimmune) |
| HuMax-EGFr | Genmab *Princeton, NJ* | head and neck cancer |
| huN901-DM1 | ImmunoGen *Cambridge, MA* | SCLC multiple myeloma |
| | | |
| ipilimumab (MDX-010) | Bristol-Myers Squibb Medarex, *Princeton,* | melanoma monotherapy leukemia, lymphoma, ovarian, prostate, renal cell cancers melanoma (MCX-010 +/- DTIC) second-line metastatic melanoma (MDX-010 disomotide/ overmotide MDX-1379) |
| | | |
| M195-bismuth 213 conjugate | Actinium Pharmaceuticals Florham Park, NJ | AML |
| M200 (volociximab) | PDL BioPharma Fremont, CA Biogen Idec Cambridge, MA | advanced solid tumors |
| MAb HeFi-1 | National Cancer Institute Bethesda, MD | lymphoma, non-Hodgkin's lymphoma |
| | | |
| MDX-060 (iratumumab) | Medarex Princeton, NJ | Hodgkin's disease, anaplastic large-cell-lymphoma |
| MDX-070 | Medarex Princeton, NJ | prostate cancer |
| | | |
| MDX-214 | Medarex *Princeton, NJ* | EGFR-expressing cancers |
| MEDI-507 siplizumab | MedImmune *Gaithersburg, MD* | T-cell lymphoma infections |
| MEDI-522 | MedImmune *Gaithersburg, MD* National Cancer Institute *Bethesda, MD* MedImmune *Gaithersburg, MD* | melanoma, prostate cancer solid tumors |
| | | |
| MORAb 003 | Morphotek *Exton, PA* | ovarian cancer |
| MORAb 009 | Morphotek *Exton, PA* | mesothelin-expressing tumors |
| | | |
| neuradiab | Bradmer Pharmaceuticals *Louisville, KY* | glioblastoma |
| nimotuzumab (Orphan Drug) | YM Biosciences *Mississauga, Ontario* | metastatic pancreatic, NSCLC |
| ocrelizumab (2nd anti-CD20) (R1594) | Genentech *South San Francisco, CA* Biogen Idec *Cambridge, MA* Roche *Nutley, NJ* | hematologic malignancies (see also autoimmune) |
| Omnitarg™ pertuzumab | Genentech *South San Francisco, CA* | ovarian cancer |
| OvaRex® oregovomab | ViRexx MAb Edmonton, Alberta | ovarian cancer |
| PAM 4 | Merck Whitehouse Station, NJ | pancreatic cancer |
| panitumumab (rHuMAb-EGFr) | Abgenix | colorectal cancer |
| Proleukin® | Chiron *Emeryville, CA* | Non-hodgkin's lymphoma |
| | | |
| PSMA | Progenics Pharmaceuticals Tarrytown, NY | prostate cancer |
| R1550 | Roche Nutley, NJ | metastatic breast cancer |
| RadioTheraCIM | YM BioSciences Mississauga, Ontario | glioma |
| RAV 12 | Raven Biotechnologies South San Francisco, CA | cancer |
| Rencarex® G250 | Wilex Munich, Germany | renal cancer |
| | | |
| Rituxan® rituximab | Genentech *South San Francisco, CA* Biogen Idec *Cambridge, MA* | indolent non-Hodgkin's lymphoma induction therapy (see also autoimmune) relapsed or refractory CLL |
| SGN-30 (Orphan Drug) | Seattle Genetics *Bothell, WA* | cutaneous anaplastic large-cell MAb lymphoma, systemic anaplastic large-cell lymphoma, Hodgkin's disease |
| SGN-33 (lintuzumab) | Seattle Genetics *Bothell, WA* | AML, myelodysplastic syndromes |
| SGN-40 | Seattle Genetics *Bothell, WA* | CLL multiple myeloma, non-Hodgkin's lymphoma |
| sibrotuzumab | Life Science Pharmaceuticals *Greenwich, CT* | colorectal, head and neck, lung cancers |
| Tarvacin™ bavituximab | Peregrine Pharmaceuticals *Tustin, CA* | solid tumors (see also infectious) |
| ticilimumab | Pfizer New York, NY | metastatic melanoma prostate cancer |
| TNX-650 | Tanox Houston, TX | Hodgkin's disease |
| Zevalin™ ibritumomab tiuxetan | National Cancer Institute *Bethesda* Biogen, | leukemia, lymphoma non-Hodgkin's lymphoma |

| Cardiovascular disease | | |
|---|---|---|
| MLN 1202 | Millennium Pharmaceuticals *Cambridge, MA* | atherosclerosis (see also autoimmune) |
| pexelizumab | Alexion Pharmaceuticals *Cheshire, CT Cheshire, CT* Procter & Gamble Pharmaceuticals *Mason, OH* | acute myocardial acute myocardial infarction, cardiopulmanory infarction, cardiopulmonary bypass |

| Diabetes and Related Conditions | | |
|---|---|---|
| anti-CD3 MAb | MacroGenics *Rockville, MD* | type-1 diabetes mellitus |
| OKT3-gamma-1 | Johnson & Johnson Pharmaceutical Research & Development | type-1 diabetes mellitus |
| TRX 4 (anti-CD3) | TolerRx *Cambridge, MA* | type-1 diabetes mellitus |

| Digestive Disorders | | |
|---|---|---|
| Remicade™ infliximab | Centocor | Crohn's disease, |
| | | |
| ABT 874 | Abbott Laboratories *Abbott Park, IL* | Crohn's disease (see also autoimmune) |
| CNTO 1275 | Centocor *Horsham, PA Horsham, PA* | Crohn's disease Phase II (see also autoimmune, skin) (610) 651-6000 |
| Humira® adalimumab | Abbott Laboratories *Abbott Park, IL* | Crohn's disease Phase III (see also autoimmune, skin) (847) 936-1189 |
| MDX-066 (CDA-1) | Medarex *Princeton, NJ* | *C. difficile disease C. difficile disease* |
| MDX-1100 | Medarex *Princeton, NJ* | ulcerative colitis |
| MLN-02 | Millennium Pharmaceuticals *Cambridge, MA* | ulcerative colitis |
| Nuvion® visilizumab | PDL BioPharma *Fremont CA* | I.V. steroid-refractory ulcerative colitis Crohn's disease |
| Tysarbi® natalizumab | Biogen Idec *Cambridge, MA* | Crohn's disease |

| Eye Conditions | | |
|---|---|---|
| golimumab | Centocor *Horsham, PA* | uveitis (see also autoimmune) |

| Genetic Disorders | | |
|---|---|---|
| Soliris™ eculizumab (Orphan Drug) | Alexion Pharmaceuticals C*heshire, CT* | paroxysmal nocturnal hemoglobinuria (PNH) |

| Neurological Disorders | | |
|---|---|---|
| RN624 | Rinat Neuroscience *South San Francisco, CA* | osteoarthritis pain |
| RN1219 | Rinat Neuroscience *South San Francisco, CA* | Alzheimer's disease |

| Respiratory Disorders | | |
|---|---|---|
| ABN 912 | Novartis Pharmaceuticals *East Hanover, NJ* | asthma, chronic obstructive pulmonary disorders (COPD) |
| ABX-IL8 | Amgen *Thousand Oaks, CA* | COPD COPD |
| AMG 317 | Amgen *Oaks, CA Thousand Oaks, CA* | asthma |
| daclizumab (anti-CD25 MAb) | Protein Design Labs *Fremont, CA Roche Nutley, NJ* | asthma (see also autoimmune) (see also autoimmune) |
| MEDI-528 anti-IL-9 MAb | MedImmune *Gaithersburg, MD* | asthma |
| mepolizumab (anti-IL5 MAb) | GlaxoSmithKline *Philadelphia, PA Rsch. Triangle Park, NC* | asthma and nasal polyposis (see also other) |
| TNX-832 | Tanox *TX Houston, TX* | respiratory diseases |
| Xolair® omalizumab | Genentech *South San Francisco, CA South San Francisco, CA* Novartis Pharmaceuticals | pediatric asthma Pediatric asthma (see also other) (see also other) |
| | | |

| Skin Disorders | | |
|---|---|---|
| Raptiva® efalizumab | Genentech XOMA | chronic moderate to severe plaque psoriasis |
| CNTO 1275 | Centocor | psoriasis see also autoimmune, digestive) |
| Humira® adalimumab | Abbott Laboratories | psoriasis see also autoimmune, digestive) |
| TRX 4 | TolerRx | psoriasis diabetes) (see also diabetes) |
| | | |

| Transplatation | | |
|---|---|---|
| ORTHOCLONEOKT®3 muromonab-CD3 | Ortho Biotech | acute kidney transplant rejection, reversal of heart and liver transplant rejection |
| Simulect® basiliximab | Novartis Pharmaceuticals | prevention of renal transplant rejection |
| Zenapax® daclizumab | Roche Protein Design Labs | prophylaxis of acute kidney transplant rejection |
| OKT3-gamma- 1 | Johnson & Johnson | renal transplant rejection (see also autoimmune, diabetes) |
| | | |

| Other | | |
|---|---|---|
| NeutroSpec™ technetium 99m Tc fanolesomab | Palatin Technologies Palatin Technologies | diagnosis of appendicitis |
| CR 0002 | CuraGen | kidney inflammation |
| denosumab (AMG 162) | Amgen | Postmenopausal osteoporosis, see also autoimmune and cancer |
| mepolizumab (anti-IL5 MAb) | GlaxoSmithKline | hypereosinophilic syndrome, eosinophilic esophagitis (see also respiratory) |
| Xolair® omalizumab | Genentech Tanox | peanut allergy(see also respiratory) |

In some aspects of the method according to the invention the method is part of a High-throughput screening (HTS). HTS as used herein refers to any automatic or semi-automatic method typically using robotics, data processing and control software, liquid handling devices, and sensitive detectors, that allow for the quickly conduct of a plurality of simultaneous tests, such as hundreds, thousands, or millions of tests. Through this process one can rapidly identify biological samples from a plurality of samples that contain antibodies against a specific bio-agent.

In some embodiments the method according to the invention is part of an ImmunoCAP assay or a modified ImmunoCAP assay, such as an assay described in e.g. WO/2008/101177, in Erwin EA, Custis NJ, Satinover SM, et al. Quantitative measurement of IgE antibodies to purified allergens using streptavidin linked to a high-capacity solid phase. J Allergy Clin Immunol 2005;l 15(5): 1029-35, or in Cavalier E, Carlisi A, Chapelle JP. [Evaluation of the analytical performance of the ImmunoCap250 (Sweden Diagnostics)]. Ann Biol Clin (Paris) 2006;64:91-4, the content of which is hereby incorporated by reference.

In some embodiments the method according to the invention is part of a simple, ready to use test, such as a doctor's office test.

### Specific embodiments of the invention

In some embodiments the antibody-binding agent according to the invention is selected from the list consisting of anti-Ig, suchs as Fc-specific or Fab specific antibodies, protein G, Protein A, and Protein H.

In some embodiments the antibody-binding agent is bound on and said first complex is formed on a suitable solid support, such as one selected from a microtiter plate and a population of magnetic beads.

In some embodiments the method according to the invention further comprises a step prior to step a) of blocking said antibody-binding agent and said solid support for unspecific binding sites with a suitable first unspecific blocking agent.

In some embodiments the method according to the invention further comprises a step prior to or simultaneous with step b) of blocking unspecific binding to said first complex with a suitable second blocking agent, such as non-labelled non-relevant immunoglobulin molecules.

As used herein, "first blocking agent" and "second blocking agent" may be used interchangeably, and only refers to the situation, wherein blocking for unspecific or non-relevant binding is performed in two separate steps, often with two different blocking agents. Most often two different blocking agents is used in the two steps, such as bovine serum albumin in the first blocking and pooled normal serum in a second blocking step. Blocking may be performed prior to or simultaneously with the addition of biological sample or labelled bio-agent.

In some embodiments, the blocking agent is normal serum comprising non-relevant immunoglobulin, such as IgG, such as normal human serum. Accordingly in some embodiments blocking is performed with more than 0.15% normal serum, such as more than 0.25% normal serum. In some embodiments blocking is performed with any solution comprising more than 1, 2, 3, 4, 5, 8, or 10 µg/ml non-relevant immunoglobulin, such as IgG, or immunoglobulin molecules comprising the Fc part.

As used herein "non-relevant immunoglobulin" refers to immunoglobulin that is different to the specific antibody that recognises the specific bio-agent being assayed according to the method.

In some embodiments the bio-agent according to the invention is a bio-diagnostic. In some embodiments the bio-agent according to the invention is a biopharmaceutical.In some embodiments bio-agent according to the invention is an immunoglobulin molecule, such as a monoclonal antibody.

In some embodiments the immunoglobulin molecule according to the invention is a single light chain subtype biopharmaceutical. In some embodiments the single light chain subtype bio-agent is a monoclonal antibody which comprises the lambda or kappa single light chain sub-type, but not both lambda and kappa single light chain sub-types.

In some embodiments the bio-agent according to the invention is either a humanised of a fully-human monoclonal antibody.

In some embodiments the biopharmaceutical according to the invention is an antibody which specifically binds TNF-alpha.

In some embodiments the biological sample according to the invention is selected from the group consisting of blood, blood serum, lymph fluid, lymph node tissue, spleen tissue, bone marrow, or an immunoglobulin enriched fraction derived from one or more of these tissues.

In some embodiments the first detectable label is selected from the group consisting of: a radio label, a fluorescent label, and a luminescent label.

In some embodiments the first detectable label is selected from the group consisting of: an enzyme label, such as NADPH, beta-galactosidase, horseradish peroxidase, glucose oxidase, alkaline phosphatase and urease and an affinity label, such as a His- His tag or a biotin label.

In some embodiments the first detectable label is a biotin label, which is measured in step c) by the application of and binding of avidin or streptavidin comprising a second detectable label.

In some embodiments the second detectable label is selected from the group consisting of: a radio label, a fluorescent label, and a luminescent label, and an enzyme label.

It is to be understood that the terms "first detectable label" and "second detectable label" may be used interchangeably. The first detectable label may or may not be directly measurable and may be dependent on the subsequent use of a second detectable label. It may be advantageous for the amplification of the signal to use a compound with a second label, which compound specifically binds the first label.

In some embodiments the contacting in any one of steps a) and/or b) is performed in a fluid phase.

In some embodiments step b) comprises a chromatographic step which enriches the second complex on a basis of molecular size or affinity.

In some embodiments step b) comprises an immuno-precipitation step.

In some embodiments the measure of the amount of antibodies which recognise the bioagent in the biological sample in step c) is performed by comparing against control samples with predetermined or known concentration of the biopharmaceutical.

In some embodiments the biological sample is contacted the antibody-binding agent, wherein the concentration of the specific antibody in the biological sample is present in a concentration of less than 10 ng/ml, such as less than 5 ng/ml, such as less than 1 ng/ml specific IgG.

In some embodiments the specific bio-agent in a form which comprises a first detectable label is contacted the first complex in a concentration less than 100 ng/ml, such as less than 80 ng/ml, such as less than 50 ng/ml.

Any combination of the above-described elements in all possible variations thereof is encompassed by the invention unless otherwise indicated herein or otherwise clearly contradicted by context.

The terms "a" and "an" and "the" and similar referents as used in the context of describing the invention are to be construed to cover both the singular and the plural, unless otherwise indicated herein or clearly contradicted by context.

Recitation of ranges of values herein are merely intended to serve as a shorthand method of referring individually to each separate value falling within the range, unless otherwise indicated herein, and each separate value is incorporated into the specification as if it were individually recited herein. Unless otherwise stated, all exact values provided herein are representative of corresponding approximate values (e.g., all exact exemplary values provided with respect to a particular factor or measurement can be considered to also provide a corresponding approximate measurement, modified by "about," where appropriate). All methods described herein can be performed in any suitable order unless otherwise indicated herein or otherwise clearly contradicted by context.

The use of any and all examples, or exemplary language (e.g., "such as") provided herein, is intended merely to better illuminate the invention and does not pose a limitation on the scope of the invention unless otherwise indicated. No language in the specification should be construed as indicating any element is essential to the practice of the invention unless as much is explicitly stated.

The citation and incorporation of patent documents herein is done for convenience only and does not reflect any view of the validity, patentability and/or enforceability of such patent documents,

The description herein of any aspect or embodiment of the invention using terms such as "comprising", "having", "including" or "containing" with reference to an element or elements is intended to provide support for a similar aspect or embodiment of the invention that "consists of", "consists essentially of", or "substantially comprises" that particular element or elements, unless otherwise stated or clearly contradicted by context (e.g., a formulation described herein as comprising a particular element should be understood as also describing a formulation consisting of that element, unless otherwise stated or clearly contradicted by context) .

This invention includes all modifications and equivalents of the subject matter recited in the aspects or claims presented herein to the maximum extent permitted by applicable law.

Numbered embodiments of the invention:
1. A method for detecting the presence of or for the measurement of the amount of specific antibodies in a biological sample derived from a subject, which antibodies recognise a specific bio-agent, said method comprising the sequential steps of:
   a) contacting the biological sample with an antibody-binding agent under conditions that allow binding of said antibodies recognising the specific bioagent, if present, to said antibody-binding agent to form a first complex;
   b) contacting said first complex, if present, with the specific bio-agent in a form which comprises a first detectable label; to form a second complex;
   c) measuring a signal from the first detectable label present in the complex formed in step b) to detect the presence of or to measure the amount of specific antibodies which recognise the bio-agent in the biological sample.
2. The method according to embodiment 1, wherein said antibody-binding agent is selected from the list consisting of anti-Ig, suchs as Fc-specific or Fab specific antibodies, protein G, Protein A, and Protein H.
3. The method according to any one of embodiments 1 or 2, wherein said antibody-binding agent is bound on and said first complex is formed on a suitable solid support, such as one selected from a microtiter plate and a population of magnetic beads.
4. The method according to embodiment 3, wherein said method further comprises a step prior to step a) of blocking said antibody-binding agent and said solid support for unspecific binding sites with a suitable first unspecific blocking agent.
5. The method according to any one of embodiments 1-4, wherein said method further comprises a step prior to or simultaneous with step b) of blocking unspecific binding to said first complex with a suitable second blocking agent, such as non-labelled non-relevant immunoglobulin molecules.
6. The method according to embodiment any one of embodiments 1-5, wherein said bioagent is a bio-diagnostic.
7. The method according to any one of embodiments 1-5, wherein said bio-agent is a biopharmaceutical.
8. The method according to any one of embodiments 1-7, wherein said bio-agent is an immunoglobulin molecule, such as a monoclonal antibody.
9. The method according to embodiment 8, wherein said immunoglobulin molecule is a single light chain subtype biopharmaceutical.
10. The method according to embodiment 9, wherein the single light chain subtype bioagent is a monoclonal antibody which comprises the lambda or kappa single light chain sub-type, but not both lambda and kappa single light chain sub-types.
11. The method according to any one of embodiments 1-10, wherein said bio-agent is either a humanised of a fully-human monoclonal antibody.
12. The method according to any one of embodiments 1-11, wherein the biopharmaceutical is an antibody which specifically binds TNF-alpha.
13. The method according to any one of embodiments 1-12, wherein the biological sample is selected from the group consisting of blood, blood serum, lymph fluid, lymph node tissue, spleen tissue, bone marrow, or an immunoglobulin enriched fraction derived from one or more of these tissues.
14. The method according to any one of embodiments 1-12, wherein the first detectable label is selected from the group consisting of: a radio label, a fluorescent label, and a luminescent label.
15. The method according to any one of embodiments 1-12, wherein the first detectable label is selected from the group consisting of: an enzyme label, such as NADPH, beta-galactosidase, horseradish peroxidase, glucose oxidase, alkaline phosphatase and urease and an affinity label, such as a His- His tag or a biotin label.
16. The method according to any one of embodiments 1-12, wherein the first detectable label is a biotin label, which is measured in step c) by the application of and binding of avidin or streptavidin comprising a second detectable label.
17. The method according to embodiment 16, wherein said second detectable label is selected from the group consisting of: a radio label, a fluorescent label, and a luminescent label, and an enzyme label.
18. The method according to any one of embodiments 1, 2, 4-17, wherein the contacting in any one of steps a) and/or b) is performed in a fluid phase.
19. The method according to any one of embodiments 1-18, wherein step b) comprises a chromatographic step which enriches the second complex on a basis of molecular size or affinity.
20. The method according to any one of embodiments 1-19, wherein step b) comprises an immuno-precipitation step.
21. The method according to any one of embodiments 1-20, wherein the measure of the amount of antibodies which recognise the bio-agent in the biological sample in step c) is performed by comparing against control samples with predetermined or known concentration of the biopharmaceutical.
22. The method according to any one of embodiments 1-21, wherein the method is performed as part of a high throughput screening.
23. The method according to any one of embodiments 1-22, wherein the method is performed as part of an immunoCAP assay.
24. A kit comprising:
   a) An antibody-binding agent;
   b) A bio-agent in a form which comprises a first detectable label,
   c) Optionally a first unspecific blocking agent and/or a second blocking agent;
   d) Optionally a suitable reagent for detection of said first or second detectable label.
25. A method of treatment of a disease in a patient being treated with a biopharmaceutical bio-agent, said method comprising performing the method according to any one of embodiments 1-5, 7-23 on a sample derived from the patient to determine whether the patient requires either an altered dosage regime of the biopharmaceutical or an alternative pharmaceutical therapy.
26. The method according to embodiment 25, wherein the method comprises periodic assessment of concentration in a biological sample derived from said patient of antibodies recognising the biopharmaceutical bio-agent.
27. The method according to embodiment 26, wherein the disease is selected from the group consisting of: rheumatoid arthritis (RA), juvenile idiopathic arthritis, ankylosing spondylitis (Bechterew's disease), inflammatory bowel diseases (Crohn's diseases and ulcerative colitis), severe psoriasis, chronic uveitis, sarcoidosis, Wegener's granulomatosis, and other diseases with inflammation as a central feature.

### EXAMPLES

### Example 1

Assay for ADA against human monoclonal Ab constructs

Principle:
1) ELISA-plate or other solid phase for fixation of Ig-binding molecules (See figure 1A). The Ig-binding molecules can be ex. anti-Ig (Fc-specific or Fab specific), protein A, protein G, protein H or similar reagents. Washing is preferably made between each new manipulation. Fixation can be non- or covalent.
   All binding sites are then blocked by an unspecific reagent not interfering with the Ig-binding capacity of the fixed Ig-binding reagent.
2) Binding of serum/plasma Ig (see figure 1B)
3) Blockade of Ig-binding sites on the fixed Ig-binding molecules, by adding non-labeled non-ADA Ig, before or together with the labelled human monoclonal Ig construct (see figure 1C).
4) Measurement of the bound label by standard methods.

### Example 2

Detailed protocol for ADA assay:
Example: EIA for ADA against Infliximab (anti-TNFa IgG)
   1) Coat: 25 µg/ml of protein G in PBS, 100 µL/well on a 96-well flat bottom plastic plate, followed by 18-36 h incubation at 4-8 C.
   2) Wash the wells manually or by a plate washer with PBS + 0.05% Tween 20
   3) Block of residual binding sites by PBS+2% BSA or Superblock (Pierce cat#37515) 2 h at room temperature or 18-24 at 4-8 C
   4) Wash the wells manually or by a plate washer with PBS + 0.05% Tween 20
   5) Sample: Serum/plasma is added at 100 µl/well diluted to ex. 0.25% in PBS+5mM EDTA+1% human serum albumin, or if further diluted, diluted in PBS+5mM EDTA+1% human serum albumin with or without 0.25% pooled normal human serum. Incubate 18-24h at 4-8 C.
   6) Wash the wells manually or by a plate washer with PBS+ 0.05% Tween 20.
   7) Ad l00 µl/well of Biotin labelled Infliximab, at ex. 40 ng/ml of PBS + 1% human serum albumin + 4% pooled normal human serum. Incubate 2 -3 h at room temperature with gentle agitation.
   8) Wash the wells manually or by a plate washer with PBS+ 0.05% Tween 20.
   9) Ad 100 µl/well of Avidin-HRPO diluted in PBS + 2% human serum albumin. Incubate for 1-1.5 h at room temperature with gentle agitation.
   10) Wash the wells manually or by a plate washer with PBS+ 0.05% Tween 20
   11) Ad 100 µl/well of TMP solution (ex. BioFX Laboratories TMBBW-1000-01) and incubate for 30+/-5 min. at room temperature.
   12) Stop the reaction by adding 100 µl/well of 2M H₂SO₄.
   13) Read the plate at 450 nm.
Note: Human serum albumin can be substituted by bovine serum albumin (BSA)

### MEASUREMENT OF ADA AGAINST INFLIXIMAB

### Comparing ADA measurements by RIA and EIA

RIA was run as described by Svenson, M. et al. (Rheumatology, 2007, 46(12): 1828-34) and the EIA essentially as described above.

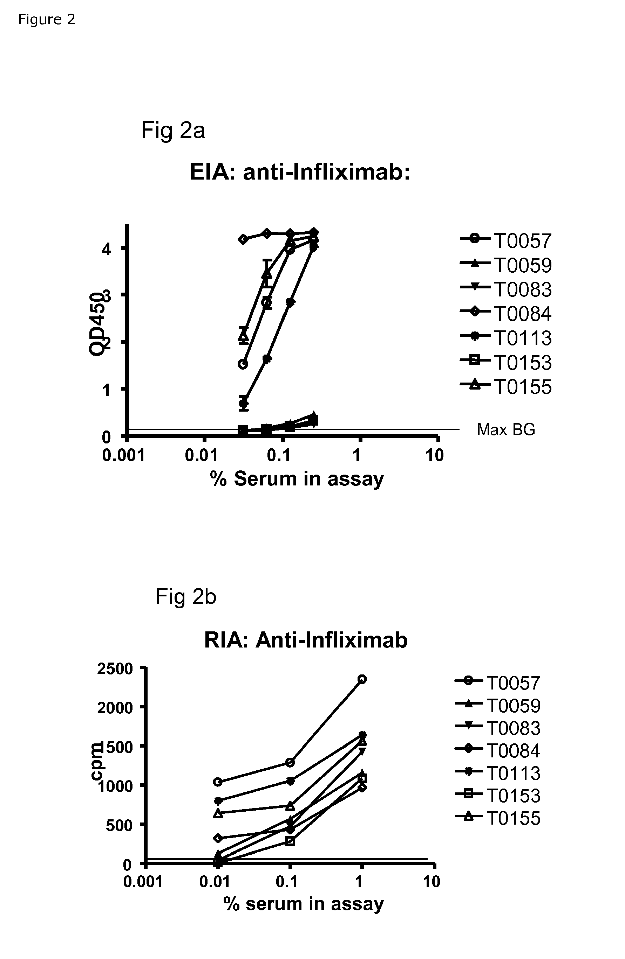

Illustrative results of running test of the same positive Infliximab ADA sera in EIA at 0.25% and in RIA at 1% concentration are show in Fig 2a and 2b. At 0.25% all sera were detected positive for ADA in the two assays, with a higher sensitivity when measured in RIA. Note the relative potency of the individual sera is different in the two assays. Of importance are the Infliximab concentrations in the two assays, because in the RIA less than 1 ng/ml but in the EIA 40ng/ml was used. This makes the RIA more sensitive to the binding avidity of the ADA, whereas in the EIA, the binding capacity will be a dominant parameter.

### Background and cross-reactivity

For measurement of background, nine sera were randomly selected among individual sera from Infliximab treated patients tested negative for Infliximab/Remicade and ADA in RIA at Biomonitor. The ADA positive sera were the ones also used in the experiment illustrated by Fig 2.

Background activity and cross-reactivity of ADA against Infliximab with two other anti-TNFa constructs was tested by substituting labelled Infliximab/Remicade with labelled Humira or Enbrel. An equivalent labelling was ensured by equal OD when detected in EIA as function of concentration when the labelled anti-TNFa was absorbed to protein G.

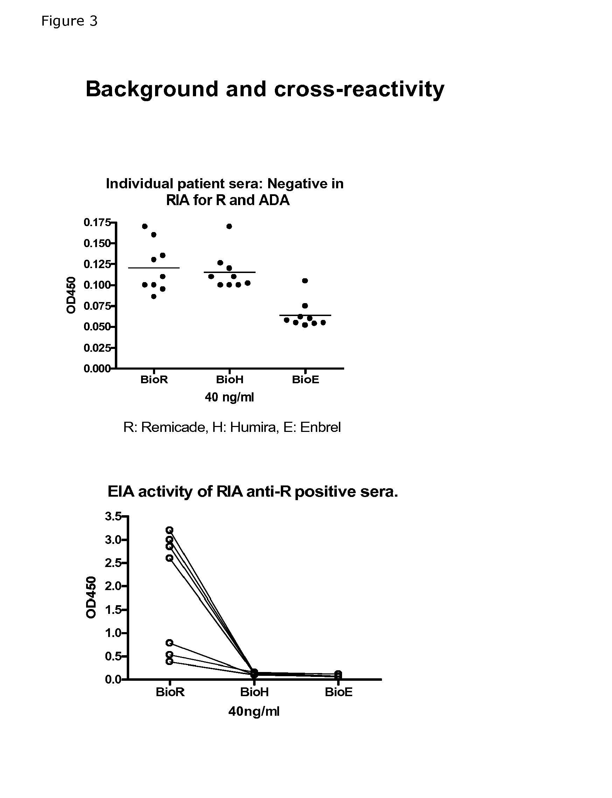

The results are illustrated by figure 3.

No cross-binding to the two other anti-TNFa constructs was observed for the Infliximab ADA, and the background OD was generally below 0.15. The observed selectivity of the Infliximab ADA in EIA is in agreement with similar test in RIA (Svenson, M. et al. Rheumatology, 2007, 46(12):1828-34).

## Claims

1. A method for detecting the presence of or for the measurement of the amount of specific antibodies in a biological sample derived from a subject, which antibodies recognise a specific bio-agent, said method comprising the sequential steps of:
a) contacting the biological sample with an antibody-binding agent under conditions that allow binding of said antibodies recognising the specific bio-agent, if present, to said antibody-binding agent to form a first complex;
b) contacting said first complex, if present, with the specific bio-agent in a form which comprises a first detectable label; to form a second complex;
c) measuring a signal from the first detectable label present in the complex formed in step b) to detect the presence of or to measure the amount of specific antibodies which recognise the bio-agent in the biological sample.

2. The method according to claim 1, wherein said antibody-binding agent is selected from the list consisting of anti-Ig, suchs as Fc-specific or Fab specific antibodies, protein G, Protein A, and Protein H.

3. The method according to any one of claims 1 or 2, wherein said antibody-binding agent is bound on and said first complex is formed on a suitable solid support, such as one selected from a microtiter plate and a population of magnetic beads.

4. The method according to claim 3, wherein said method further comprises a step prior to step a) of blocking said antibody-binding agent and said solid support for unspecific binding sites with a suitable first unspecific blocking agent.

5. The method according to any one of claims 1-4, wherein said method further comprises a step prior to or simultaneous with step b) of blocking unspecific binding to said first complex with a suitable second blocking agent, such as non-labelled non-relevant immunoglobulin molecules.

6. The method according to claim any one of claims 1-5, wherein said bio-agent is a biodiagnostic.

7. The method according to any one of claims 1-5, wherein said bio-agent is a biopharmaceutical.

8. The method according to any one of claims 1-7, wherein said bio-agent is an immunoglobulin molecule, such as a monoclonal antibody.

9. The method according to any one of claims 1-8, wherein said bio-agent is either a humanised of a fully-human monoclonal antibody.

10. The method according to any one of claims 1-9, wherein the first detectable label is selected from the group consisting of: an enzyme label, such as NADPH, beta-galactosidase, horseradish peroxidase, glucose oxidase, alkaline phosphatase and urease and an affinity label, such as a His- His tag or a biotin label.

11. The method according to any one of claims 1-10, wherein the first detectable label is a biotin label, which is measured in step c) by the application of and binding of avidin or streptavidin comprising a second detectable label.

12. The method according to any one of claims 1-11, wherein the measure of the amount of antibodies which recognise the bio-agent in the biological sample in step c) is performed by comparing against control samples with predetermined or known concentration of the biopharmaceutical.

13. The method according to any one of claims 1-12, wherein the method is performed as part of a high throughput screening.

14. The method according to any one of claims 1-13, wherein the method is performed as part of an immunoCAP assay.

15. A method of treatment of a disease in a patient being treated with a biopharmaceutical bio-agent, said method comprising performing the method according to any one of claims 1-5, 7-14 on a sample derived from the patient to determine whether the patient requires either an altered dosage regime of the biopharmaceutical or an alternative pharmaceutical therapy.
